# EUROPÄISCHE PATENTSCHRIFT

(11) **EP 2 822 921 B1**
(45) Veröffentlichungstag und Bekanntmachung des Hinweises auf die Patenterteilung: **20.06.2018**
(21) Anmeldenummer: 13707637.8
(22) Anmeldetag: 06.03.2013
(51) Int. Cl.: C07C 29/141

(54) **VERFAHREN ZUR WÄRMEINTEGRATION BEI DER HYDRIERUNG UND DESTILLATION VON C3-C20 ALDEHYDEN**
METHOD FOR THERMAL INTEGRATION IN THE HYDROGENATION AND DESTILLATION OF C3-C20 ALDEHYDES
PROCÉDÉ D'INTEGRATION THERMIQUE LORS DE L'HYDROGÉNATION ET DE LA DISTILLATION D'ALDÉHYDES EN C3-C20

(30) Priorität: 07.03.2012 EP 12158380
(43) Veröffentlichungstag der Anmeldung: 14.01.2015
(73) Patentinhaber: BASF SE, 67056 Ludwigshafen am Rhein (DE)
(72) Erfinder: KROKOSZINSKI, Roland, 67273 Weisenheim a.Berg (DE); WALCZUCH, Karl-Heinz, 67071 Ludwigshafen (DE)
(74) Vertreter: BASF IP Association
(86) Internationale Anmeldenummer: PCT/EP2013/054467
(87) Internationale Veröffentlichungsnummer: WO 2013/131936

(56) Entgegenhaltungen:
- US-A- 4 451 677

## Beschreibung

Die vorliegende Erfindung betrifft ein Verfahren zur Wärmeintegration bei der Herstellung von gesättigten C₃-C₂₀-Alkoholen, bei dem man einen Hydrierzulauf, der wenigstens einen C₃-C₂₀-Aldehyd enthält, in einer Hydrierzone in Gegenwart eines wasserstoffhaltigen Gases hydriert, aus der Hydrierzone einen Austrag entnimmt und einer Destillation in wenigstens einer Destillationskolonne unterzieht, wobei eine an gesättigten C₃-C₂₀-Alkoholen angereicherte Fraktion erhalten wird.

Die katalytische Hydrierung von Aldehyden zur Gewinnung von Alkoholen ist ein seit Jahrzehnten ausgeübtes großtechnisches Verfahren, bei dem eine Vielzahl von Katalysatoren, die im Allgemeinen aus Elementen der VI. bis VIII. sowie der I. Nebengruppe des Periodensystems, insbesondere aus den Elementen Chrom, Mangan, Eisen, Kobalt, Nickel und/oder Kupfer, bestehen, eingesetzt werden. Solche Katalysatoren sind z. B. in der DE-A 32 28 881, DE-A 26 28 987 und DE-A 24 45 303 beschrieben. Die nach diesen Verfahren hergestellten Alkohole finden breite Verwendung, z. B. als Lösungsmittel oder als Weichmacheralkohole.

Bei der Hydroformylierung von Propen resultiert ein n-/iso-Butyraldehyd-haltiges Gemisch, das einer Weiterverarbeitung zu mehreren Wertprodukten unterzogen werden kann. Das n-/iso-Butyraldehyd-haltige Gemisch kann, gegebenenfalls nach einer destillativen Auftrennung in n-Butyraldehyd und iso-Butyraldehyd, einer Hydrierung unter Erhalt von n-Butanol und von iso-Butanol unterzogen werden. Die Alkohole werden in der Regel zusätzlich einer destillativen Reinigung unterzogen.

n-Butyraldehyd ist auch ein wichtiges Zwischenprodukt bei der Herstellung von 2-Ethylhexanol durch Aldoladdition, Dehydratisierung und Hydrierung. Dabei wird die "Aldolkondensation" d. h. die Kombination aus Aldoladdition zum β-Hydroxy-Aldehydaddukt und anschließender Wasserabspaltung zu den betreffenden α,β-ungesättigten Aldehyden im Folgenden auch als "Enalisierung" bezeichnet.

2-Ethylhexanol (2-EH) dient unter Anderem zur Herstellung von Phthalat-Weichmacher und von Acrylat-Ester, die in Klebstoffen und Oberflächenbeschichtung, wie Acrylfarben oder Druckfarben, und als Imprägniermittel verwendet werden.

Die Hydrierung von niederen Aldehyden ist eine exotherme Reaktion. Zur Abführung der Reaktionswärme muss das Reaktionsgemisch daher einer Kühlung unterzogen werden, z. B. indem man es durch einen Wärmeaustauscher leitet und die Reaktionswärme auf ein Kühlmedium, z. B. einen Kühlwasserstrom oder Luft, überträgt.

Bei der Hydrierung von Aldehyden, insbesondere bei hohen Hydriertemperaturen, laufen neben der erwünschten Hydrierung des Aldehyds zum Alkohol verschiedene unerwünschte Nebenreaktionen, wie Acetalisierungen oder Aldolisierungen, die Tischtschenko-Reaktion oder die Etherbildung, ab. Daher wird das Hydrierprodukt in der Regel einer Aufarbeitung, z. B. einer ein- oder mehrstufigen Destillation, unterzogen, um den betreffenden Alkohol in der gewünschten Reinheit zu erhalten. Bei der destillativen Auftrennung des Rohalkohols aus der Aldehydhydrierung wird das Reaktionsgemisch im Sumpf der Destillationskolonne(n) verdampft und am Kopf wieder kondensiert. Die dem Sumpf zugeführte Verdampfungsenergie wird am Kopf über Wärmetauscher wieder abgeführt, wobei als Kühlmittel, wie bei der Hydrierung, üblicherweise Wasser oder Luft verwendet werden.

Die WO 01/87809 beschreibt ein Verfahren zur Herstellung von gesättigten C₃-C₂₀-Alkoholen, bei dem man einen flüssigen Hydrierzulauf, der wenigstens einen C₃-C₂₀-Aldehyd enthält, in Gegenwart eines wasserstoffhaltigen Gases über ein Bett eines Hydrierkatalysators leitet, wobei man dem Hydrierzulauf eine darin homogen lösliche Menge einer salzartigen Base zusetzt. Durch den Basenzusatz werden Nebenreaktionen, wie die Acetalisierung, Aldolisierung, Tischtschenko-Reaktion oder Etherbildung zurückgedrängt. Das Hydrierungsprodukt kann einer destillativen Auftrennung unterzogen werden.

Die bekannten Verfahren zur Herstellung von gesättigten Alkoholen durch Hydrierung der entsprechenden Aldehyde und anschließende Destillation des Rohalkoholgemischs sind im Hinblick auf ihre Energieausnutzung verbesserungswürdig. Wird bei der Kühlung der Hydrierreaktion und/oder der Kondensation des Kopfprodukts der Destillation Energie lediglich auf ein Kühlmedium übertragen und aus dem Prozess ausgeschleust, so geht eine große Energiemenge ungenutzt verloren. Aber auch bei Einsatz eines klassischen Wärmeverbunds, bei dem die Hydrier- und/oder die Kondensationswärme in einem ersten Wärmetauscher auf ein Wärmeträgermedium übertragen und nach Transport an den Ort eines energieverbrauchenden Prozesses in einem zweiten Wärmetauscher auf einen Stoffstrom dieses Prozesses übertragen wird, ist noch verbesserungswürdig.

Der vorliegenden Erfindung liegt die Aufgabe zu Grunde, ein Verfahren zur Herstellung von gesättigten C₃-C₂₀-Alkoholen aus C₃-C₂₀-Aldehyden zur Verfügung zu stellen, bei dem man die Hydrierwärme und/oder die bei der Destillation der Alkohole anfallende Kondensationswärme effektiv und einfach abführen und mit möglichst hoher Energieausnutzung wieder einsetzen kann.
Diese Aufgabe wird erfindungsgemäß durch ein Verfahren zur Wärmeintegration bei der Herstellung von gesättigten C₃-C₂₀-Alkoholen gelöst, bei dem man einen Hydrierzulauf, der wenigstens einen C₃-C₂₀-Aldehyd enthält, in einer Hydrierzone in Gegenwart eines wasserstoffhaltigen Gases hydriert, aus der Hydrierzone einen Austrag entnimmt und einer Destillation in wenigstens einer Destillationskolonne unterzieht, wobei eine an gesättigten C₃-C₂₀-Alkoholen angereicherte Fraktion erhalten wird, wobei man
- aus der Hydrierzone einen flüssigen Strom S1) entnimmt, diesem Strom Wärme entzieht und ihn anschließend in die Hydrierungszone zurückführt, und/oder
- am Kopf wenigstens einer der Destillationskolonnen einen gasförmigen Strom S2) entnimmt, diesem Strom Wärme entzieht und ihn gegebenenfalls anschließend in die Destillation zurückführt,
wobei die Wärme dem Strom S1) und/oder dem Strom S2) durch indirekten Wärmeaustausch entzogen und ohne den Einsatz eines Hilfsmediums zur Wärmeübertragung auf einen zu erwärmenden Stoffstrom übertragen wird, wobei man ein Aldehydgemisch, das wenigstens zwei C₃-C₂₀-Aldehyde gleicher Kohlenstoffatomanzahl aufweist, einer distillativen Auftrennung unter Erhalt einer Fraktion unterzieht, welche die Hauptmenge eines der C₃-C₂₀-Aldehyde enthält und diese Fraktion als Hydrierzulauf einsetzt, und man die dem Strom S1) und/oder die dem Strom S2) entzogenen Wärme zur Erwärmung des destillativ aufzutrennenden Aldehydgemischs einsetzt. Eine spezielle Ausführung der Erfindung ist ein Verfahren zur Wärmeintegration bei der Herstellung von n-Butanol und/oder iso-Butanol, bei dem man
a) ein n-/iso-Butyraldehydgemisch einer destillativen Auftrennung unter Erhalt einer die Hauptmenge des n-Butyraldehyd enthaltenden Fraktion und einer die Hauptmenge des iso-Butyraldehyd enthaltenden Fraktion unterzieht,
b1) den die Hauptmenge des n-Butyraldehyd enthaltenden Strom aus Schritt a) in einer Hydrierungszone H1) in Gegenwart von Wasserstoff einer Hydrierung unterzieht,
c1) aus der Hydrierungszone H1) einen flüssigen Strom S1c1) entnimmt, diesem Strom Wärme entzieht, indem man ihn gemeinsam mit dem in Schritt a) aufzutrennenden n-/iso-Butyraldehydgemisch durch einen Wärmeaustauscher führt und ihn anschließend in die Hydrierzonezone H1) zurückführt,
d1) aus der Hydrierungszone H1) einen Austrag entnimmt und einer Destillation in wenigstens einer Destillationskolonne unterzieht, wobei eine an n-Butanol angereicherte Fraktion erhalten wird,
e1) am Kopf wenigstens einer der Destillationskolonnen einen gasförmigen Strom S2e1) entnimmt, diesem Strom Wärme entzieht, indem man ihn gemeinsam mit dem in Schritt a) aufzutrennenden n-/iso-Butyraldehydgemisch durch einen Wärmeaustauscher führt und ihn gegebenenfalls anschließend in die Destillation zurückführt,
   und/oder
b2) den die Hauptmenge des iso-Butyraldehyd enthaltenden Strom aus Schritt a) in einer Hydrierungszone H2) in Gegenwart von Wasserstoff einer Hydrierung unterzieht,
c2) aus der Hydrierungszone H2) einen flüssigen Strom S1c2) entnimmt, diesem Strom Wärme entzieht, indem man ihn gemeinsam mit dem in Schritt a) aufzutrennenden n-/iso-Butyraldehydgemisch durch einen Wärmeaustauscher führt und ihn anschließend in die Hydrierzonezone H2) zurückführt,
d2) aus der Hydrierungszone H2) einen Austrag entnimmt und einer Destillation in wenigstens einer Destillationskolonne unterzieht, wobei eine an iso-Butanol angereicherte Fraktion erhalten wird,
e2) am Kopf wenigstens einer der Destillationskolonnen einen gasförmigen Strom S2e2) entnimmt, diesem Strom Wärme entzieht, indem man ihn gemeinsam mit dem in Schritt a) aufzutrennenden n-/iso-Butyraldehydgemisch durch einen Wärmeaustauscher führt und ihn gegebenenfalls anschließend in die Destillation zurückführt,
mit der Maßgabe, dass wenigstens einer der Schritte c1), e1), c2) oder e2) zwingend vorgesehen ist.

In einer weiteren speziellen Ausführungsform umfasst das erfindungsgemäße Verfahren zur Wärmeintegration die folgenden Schritte:
- Bereitstellung wenigstens eines n-/iso-Butyraldehydgemischs durch Hydroformylierung eines Propen-haltigen Ausgangsmaterials,
- destillative Auftrennung des n-/iso-Butyraldehydgemischs unter Erhalt einer die Hauptmenge des n-Butyraldehyd enthaltenden Fraktion und einer die Hauptmenge des iso-Butyraldehyd enthaltenden Fraktion,
- Aufteilung der die Hauptmenge des n-Butyraldehyd enthaltenden Fraktion in zwei Teilfraktionen,
- Hydrierung der ersten Teilfraktion in einer Hydrierzone in Gegenwart eines wasserstoffhaltigen Gases und Destillation des der Hydrierzone entnommenen Austrags in wenigstens einer Destillationskolonne, wobei eine an n-Butanol angereicherte Fraktion erhalten wird,
- Unterziehen der zweiten Teilfraktion einer Enalisierungsreaktion, katalytische Hydrierung des bei der Enalisierungsreaktion erhaltenen Produktgemischs und Destillation des Hydrierprodukts unter Erhalt einer an 2-Ethylhexanol angereicherten Fraktion,
wobei man
- aus der Hydrierzone zur Hydrierung der ersten Teilfraktion einen flüssigen Strom S1) entnimmt, diesem Strom Wärme entzieht, indem man ihn gemeinsam mit dem aufzutrennenden n-/iso-Butyraldehydgemisch durch einen Wärmeaustauscher führt und ihn anschließend in die Hydrierungszone zurückführt, und/oder
- am Kopf wenigstens einer Destillationskolonne zur Destillation des Austrags aus der Hydrierzone einen gasförmigen Strom S2) entnimmt, diesem Strom Wärme entzieht, indem man ihn gemeinsam mit dem aufzutrennenden n-/iso-Butyraldehydgemisch durch einen Wärmeaustauscher führt und ihn gegebenenfalls anschließend in die Destillation zurückführt, und/oder
- bei der Hydrierung des Produkts der Enalisierungsreaktion einen flüssigen Strom S3) entnimmt und diesem Strom S3) Wärme entzieht, indem man ihn gemeinsam mit dem aufzutrennenden n-/iso-Butyraldehydgemisch durch einen Wärmeaustauscher führt und den Strom S3) anschließend in die Hydrierung des Produkts der Enalisierungsreaktion zurückführt.

Sofern keine speziellen Angaben gemacht werden, wird bezüglich geeigneter und bevorzugter Ausführungsformen der Schritte a), b1) bis e1) und b2) bis e2) des Verfahrens zur Wärmeintegration bei der Herstellung von n-Butanol und/oder iso-Butanol auf die folgenden Angaben zu geeigneten und bevorzugten Ausführungsformen des erfindungsgemäßen Verfahrens zur Wärmeintegration bei der Herstellung von gesättigten C₃-C₂₀-Alkoholen Bezug genommen.

Das erfindungsgemäße Verfahren wird vorzugsweise kontinuierlich durchgeführt.

Beim indirekten Wärmeaustausch findet die Übertragung von Wärme von einem Fluid mit höherer Temperatur auf ein kälteres Fluid über eine die fluiden Phasen trennende Wand statt. Zur indirekten Übertragung von Wärmeenergie von einem Fluidstrom auf einen anderen kann man die beiden Fluide durch einen Wärmeaustauscher führen. Erfindungsgemäß wird zum indirekten Wärmeaustausch ein üblicher Wärmeaustauscher (auch Wärmetauscher oder Wärmeübertrager) eingesetzt. Der örtliche Temperaturverlauf beim Wärmeaustausch hängt stark davon ab, in welche Richtung die Strömung des warmen und des kalten Mediums erfolgt, wobei man drei grundsätzliche Fälle unterscheidet:
- Parallel- oder Gleichstrom: Warmes und kaltes Medium strömen entlang der Wärmeaustauschfläche in gleicher Richtung,
- Gegenstrom: Warmes und kaltes Medium strömen beiderseits ihrer Trennwand in entgegengesetzter Richtung,
- Kreuz- und Querstrom: Der das warme Medium enthaltende Strom strömt senkrecht auf die Trennwand, also quer zum kalten Medium.

Daneben ist eine Reihe von Kombinationen dieser Grundarten möglich. So wird bei Rohrbündelreaktoren häufig der Kreuzgegenstrom angewendet, bei dem durch Umlenkbleche das zu erwärmende und das zu kühlende Medium sinusförmig geleitet werden.

Geeignete Wärmeaustauscher sind die üblichen, dem Fachmann bekannten Vorrichtungen, in welchen Wärme von einem Medium auf ein anderes übertragen wird. Typische Ausführungsformen von Wärmeaustauschern sind Kondensatoren und Verdampfer. Die erfindungsgemäß eingesetzten Wärmeaustauscher können in üblichen Bauarten ausgeführt sein, wie z. B. Platten-, Ringnut-, Rippenrohr-, Lamellen-, Doppelrohr-, Rohrbündel-, Spaltrohr-, Teller-, Spiral-, Block-, Kratz-, Schnecken-, Wendel-, Wirbelschicht-, Kerzen-, gekühlte Umwälz- sowie Zwei- und Dreirohrschlangenaustauscher.

Nach einer bevorzugten Ausführungsform wird die dem Strom S1) und/oder S2) entzogene Wärme in einem wärmeverbrauchenden Schritt des erfindungsgemäßen Verfahrens zur Herstellung von gesättigten C₃-C₂₀-Alkoholen eingesetzt. Dazu zählen neben der Hydrierung wenigstens eines C₃-C₂₀-Aldehyds und der Destillation des Austrags aus der Hydrierzone auch weitere vorausgehende und/oder nachfolgende Reaktions- und/oder Aufarbeitungsschritte.

Erfindungsgemäß werden die Ströme S1), S2) und S3, soweit vorhanden, an den Ort des Wärmeverbrauchs geführt, ohne die enthaltene Wärme zwischenzeitlich auf ein Hilfsmedium zu übertragen. Dies kann z. B. in üblichen, gegebenenfalls wärmeisolierten und/oder druckfest ausgerüsteten Rohrleitungen, erfolgen. Ein wesentlicher wärmeverbrauchender Schritt des erfindungsgemäßen Verfahrens ist die destillative Auftrennung eines C₃-C₂₀-Aldehydgemischs zur Bereitstellung des Hydrierzulaufs. Speziell handelt es sich bei dem wärmeverbrauchenden Schritt um die destillative Auftrennung eines C₃-C₂₀-Aldehydgemischs, das wenigstens zwei C₃-C₂₀-Alkanale gleicher Kohlenstoffatomanzahl aufweist. Wie im Folgenden näher ausgeführt, sind solche Aldehydgemische durch Hydroformylierung der entsprechenden ein Kohlenstoffatom weniger aufweisenden Olefine erhältlich.

Bevorzugt wird der Strom S1) ohne Abtrennung einer stofflichen Komponente in die Hydrierzone zurückgeführt. Das heißt, dass von dem aus der Hydrierzone entnommenen Strom S1) vor dessen Rückführung in die Hydrierungszone keine stoffliche Komponente, z. B. in Form von Produkten, Edukten etc., abgetrennt wird.

Bevorzugt weist der in die Hydrierzone zurückgeführte Strom S1) eine Temperatur auf, die etwa 3 bis 30 °C, bevorzugt 5 bis 25 °C, unterhalb der Temperatur in der Reaktionszone liegt.

Bevorzugt wird der Strom S2) gasförmig entnommen und beim Wärmeaustausch kondensiert. Somit kann vorteilhafterweise die Kondensationswärme für die Wärmeintegration genutzt werden.

Bevorzugt wird der Strom S3) ohne Abtrennung einer stofflichen Komponente in die Hydrierzone zurückgeführt. Das heißt, dass von dem bei der Hydrierung des Produkts der Enalisierungsreaktion entnommenen Strom S3) vor dessen Rückführung in die Hydrierungszone keine stoffliche Komponente, z. B. in Form von Produkten, Edukten etc., abgetrennt wird.

Bevorzugt weist der in die Hydrierung des Produkts der Enalisierungsreaktion zurückgeführte Strom S3) eine Temperatur auf, die etwa 3 bis 30 °C, bevorzugt 5 bis 25 °C, unterhalb der Temperatur in der Reaktionszone liegt.

### Hydrierzulauf

Bevorzugt wird der Hydrierzulauf, der wenigstens einen C₃-C₂₀-Aldehyd enthält, der Hydrierzone flüssig zugeführt.

Der Hydrierzulauf kann aus einem Aldehyd oder aus mehreren Aldehyden bestehen.

Bei den zu hydrierenden Aldehyden handelt es sich vorzugsweise um aliphatische C₃-bis C₂₀-Aldehyde, insbesondere C₃- bis C₁₅-Aldehyde. Die Aldehyde können geradkettig oder verzweigt sein und zusätzlich Doppelbindungen im Molekül enthalten. Prinzipiell unterliegen die in dem erfindungsgemäßen Verfahren einsetzbaren Aldehyde keinen Beschränkungen.

Bevorzugt ist der C₃-C₂₀-Aldehyd ausgewählt unter Propionaldehyd, n-Butyraldehyd, iso-Butyraldehyd, Pentanal, Hexanal, Heptanal, 2-Ethylhexanal, 2-Ethylhexenal, Nonanal, Nonenal, Decanal, Decenal sowie den Hydroformylierungsprodukten von Trimerpropylen, Tetramerpropylen, Dimerbuten oder Trimerbuten.

Besonders bevorzugt ist der C₃-C₂₀-Aldehyd ausgewählt unter n-Butyraldehyd, iso-Butyraldehyd und Mischungen davon. Speziell wird n-Butyraldehyd eingesetzt.

Gewünschtenfalls können die Aldehyde als Lösung in einem inerten Verdünnungsmittel eingesetzt werden. Geeignete inerte Verdünnungsmittel sind beispielsweise Kohlenwasserstoffe, Ether, wie Diethylether, oder Alkohole. Besonders bevorzugt werden Alkohole als Verdünnungsmittel verwendet, insbesondere derjenige Alkohol, der das Hydrierungsprodukt des zu hydrierenden Aldehyds ist. In einer speziellen Ausführungsform wird hierzu eine Teilmenge des Austrags aus der Hydrierungszone zurückgeführt und mit dem Hydrierzulauf vermischt. Falls verwendet, wird das inerte Verdünnungsmittel vorzugsweise in einer Menge von 0,1 bis 100 Gewichtsteilen, insbesondere 1 bis 50 Gewichtsteilen und besonders bevorzugt 5 bis 20 Gewichtsteilen, bezogen auf 1 Gewichtsteil eingesetzten Aldehyd, verwendet.

Der Hydrierzulauf kann Spuren von Wasser enthalten, z. B. in der Größenordnung von 1 ppm bis 4 Gew.-%, die durch die Edukte in den vorhergegangenen Synthesestufen eingeführt, durch Kondensationsreaktionen gebildet worden oder dem Hydrierzulauf bewusst zugesetzt worden sind. Diese Spuren von Wasser sind unkritisch für das erfindungsgemäße Verfahren, speziell solange das Wasser in der organischen Phase gelöst bleibt.

### Hydroformylierung

Die Bereitstellung von C₃-C₂₀-Aldehyden für das erfindungsgemäße Verfahren erfolgt vorzugsweise durch Hydroformylierung entsprechender C₂-C₁₉-Olefin-Ausgangsmaterialien. Derartige Verfahren sind dem Fachmann bekannt. So beschreibt z. B. die EP 0 648 730 A1 die Herstellung von Hydroformylierungsprodukten des Propens, indem man einen Propenstrom zusammen mit Kohlenmonoxid und Wasserstoff in einen Hydroformylierungsreaktor eingespeist und in Gegenwart eines Hydroformylierungskatalysators umgesetzt.

Geeignete Hydroformylierungskatalysatoren sind dem Fachmann ebenfalls bekannt. Bevorzugt wird zur Hydroformylierung ein homogen im Reaktionsmedium der Hydroformylierungsreaktion löslicher Rhodium-Komplex-Katalysator eingesetzt, der eine oder mehrere Organophosphorverbindung(en) als Liganden aufweist. Geeignete Liganden sind Phosphin-Liganden aus der Klasse der Triarylphosphine, C₁-C₆-Alkyldiarylphosphine oder Arylalkyldiphosphine und insbesondere Triphenylphosphin. Des Weiteren geeignete Rhodium-Komplex-Katalysatoren mit sterisch gehinderten Phosphit-Liganden sind z. B. in US-A 4 668 651, US-A 4 748 261, US-A 4 769 498, US-A 4 774 361, US-A 4 835 299, US-A 4 885 401, US-A 5 059 710, US-A 5 113 022, US-A 5 179 055, US-A 5 260 491, US-A 5 264 616, US-A 5 288 918, US-A 5 360 938, EP-A 472 071 und EP-A 518 241 beschrieben.

Die Abtrennung des Hydroformylierungsproduktes vom Austrag aus der Reaktionszone kann auf verschiedene Weise erfolgen. Es kann zum Beispiel ein Hydroformylierungsverfahren mit Flüssigaustrag zum Einsatz kommen, wie es z. B. in der US-A 4 148 830, EP-A 16 286, EP-A 188 246 oder EP-A 423 769 beschrieben ist. Bevorzugt ist ein Flüssigaustragverfahren, bei dem der im Wesentlichen, bis auf das im Überschuss zur Hydroformylierung eingesetzte Synthesegas, flüssige Austrag aus der Reaktionszone entspannt wird, wobei infolge der Druckerniedrigung der Austrag in eine Flüssigphase, die im Wesentlichen aus hochsiedenden Nebenprodukten, dem homogen gelösten Hydroformylierungskatalysator, einem Teil des Hydroformylierungsprodukts und gelöstem, nicht umgesetztem Propylen und Propan besteht, und eine Gasphase, die im Wesentlichen aus Hydroformylierungsprodukt, nicht umgesetztem Propylen und Propan sowie nicht umgesetztem Kohlenmonoxid und Wasserstoff sowie Inerten (z. B. N2, CO2, Methan) besteht, aufgetrennt wird. Die Flüssigphase kann, gegebenenfalls nach weiterer Abtrennung darin enthaltener Produktaldehyde, als Rückführstrom wieder in den Reaktor geleitet werden. Durch zumindest partielle Kondensation der Gasphase wird das rohe Hydroformylierungsprodukt erhalten. Die bei der Kondensation zurückbleibende Gasphase wird ganz oder teilweise in die Reaktionszone zurückgeführt. Mit Vorteil kann die in der Entspannungsstufe primär anfallende Gas- und Flüssigphase nach dem in der WO 97/07086 beschriebenen Verfahren aufgearbeitet werden. Zu diesem Zweck wird die Flüssigphase erwärmt und in den oberen Bereich einer Kolonne eingeleitet, während die Gasphase in den Sumpf der Kolonne eingeleitet wird. Flüssigphase und Gasphase werden dadurch im Gegenstrom geführt. Durch den innigen Kontakt der Gasphase mit der Flüssigphase werden die in der Flüssigphase vorhandenen Restmengen an Hydroformylierungsprodukt, nicht umgesetztem Propylen und Propan in die Gasphase transferiert, so dass der die Kolonne am Kopf verlassende Gasstrom im Vergleich zu dem am unteren Ende der Kolonne eingeführten Gasstrom an Hydroformylierungsprodukt sowie nicht umgesetztem Propylen und Propan angereichert ist.

Alternativ kann man nach dem sogenannten Kreisgasverfahren verfahren, bei dem aus dem Gasraum des Hydroformylierungsreaktors ein Gasstrom abgezogen wird. Dieser Gasstrom besteht im Wesentlichen aus Synthesegas, nicht umgesetztem Propylen und Propan, wobei nach Maßgabe des Dampfdrucks im Hydroformylierungsreaktor das bei der Hydroformylierungsreaktion gebildete Hydroformylierungsprodukt mitgeführt wird. Aus dem Gasstrom wird das mitgeführte rohe Hydroformylierungsprodukt z. B. durch Abkühlen auskondensiert, und der vom Flüssiganteil befreite Gasstrom wird zurück in den Hydroformylierungsreaktor geführt. Das im auskondensierten rohen Hydroformylierungsprodukt gelöst enthaltene, nicht umgesetzte Propylen und Propan kann dann, wie beschrieben, z. B. in einer Entgasungskolonne freigesetzt werden.

### Aldehyddestillation

Bei der Herstellung der Aldehyde durch Hydroformylierung fallen vielfach Produktgemische an, die wenigstens zwei isomere Aldehyde mit gleicher Kohlenstoffatomanzahl enthalten. Häufig ist für die weitere Verwendung der nach dem erfindungsgemäßen Verfahren erhaltenen gesättigten C₃-C₂₀-Alkohole jedoch ein Isomerengemisch unerwünscht. Dies gilt speziell auch für den Fall, dass zur Bereitstellung des Hydrierzulaufs ein n-/iso-Butyraldehydgemisch aus der Propenhydroformylierung eingesetzt wird und im Rahmen eines Verbundprozesses ein Teil des n-Butyraldehyds zu 2-Ethylhexanol weiterverarbeitet werden soll. Hier ist der Einsatz von Aldehydgemischen, die iso-Butyraldehyd enthalten, nicht erwünscht, da iso-Butyraldehyd zur Bildung von 2-Ethyl-4-methyl-pentanol führt, welches sich destillativ nicht ökonomisch von 2-Ethylhexanol abtrennen lässt.

In vielen Fällen lassen sich die isomeren C₃-C₂₀-Aldehyde leichter auftrennen als die daraus nach der Hydrierung erhaltenen isomeren C₃-C₂₀-Alkohole. Dies gilt speziell für Gemische aus n-Butyraldehyd und iso-Butyraldehyd, wie ein Vergleich der Siedepunkte unter Normalbedingungen zeigt: n-Butyraldehyd: 75 °C, iso-Butyraldehyd: 63 °C, n-Butanol: 117,7 °C, iso-Butanol (2-Methyl-1-propanol): 108 °C.

Der Vorteil der Hydrierung mit destilliertem Aldehydzulauf ist wesentlich auch die geringere Nebenproduktbildung und längere Katalysatorstandzeit in der Hydrierung.

In einer bevorzugten Ausführungsform des erfindungsgemäßen Verfahrens wird daher zur Bereitstellung des Hydrierzulaufs ein Aldehydgemisch, das wenigstens zwei C₃-C₂₀-Aldehyde gleicher Kohlenstoffatomanzahl aufweist, einer destillativen Auftrennung unter Erhalt einer Fraktion unterzogen, welche die Hauptmenge eines der C₃-C₂₀-Aldehyde enthält, und diese Fraktion dann als Hydrierzulauf eingesetzt.

Gewünschtenfalls erfolgt die destillative Auftrennung unter Erhalt von wenigstens zwei Fraktionen, wobei jede die Hauptmenge eines der isomeren C₃-C₂₀-Aldehyde enthält. Es ist dann möglich, nur eine oder mehrere dieser Fraktionen jeweils separat einer Hydrierung und destillativen Auftrennung nach dem erfindungsgemäßen Verfahren zu unterziehen. Dabei kann entweder nur bei der Hydrierung und Destillation eines der isomeren Aldehyde oder bei der Hydrierung und Destillation mehrerer der isomeren Aldehyde ein Strom S1) und/oder ein Strom S2) entnommen werden. Die Ströme S1) und S2) können unabhängig voneinander auf gleiche oder verschiedene zu erwärmende Stoffströme übertragen werden. In einer speziellen Ausführungsform werden alle entnommenen Ströme S1) und S2) zur Erwärmung des destillativ aufzutrennenden Aldehydgemischs eingesetzt.

In einer speziell bevorzugten Ausführungsform des erfindungsgemäßen Verfahrens wird zur Bereitstellung des Hydrierzulaufs ein n-/iso-Butyraldehydgemisch einer destillativen Auftrennung unter Erhalt einer die Hauptmenge des n-Butyraldehyds enthaltenden Fraktion und einer die Hauptmenge des iso-Butyraldehyds enthaltenden Fraktion unterzogen und die die Hauptmenge des n-Butyraldehyds enthaltende Fraktion oder die die Hauptmenge des iso-Butyraldehyds enthaltende Fraktion als Hydrierzulauf eingesetzt.

Selbstverständlich ist es ebenfalls möglich, sowohl die n-Butyraldehyd enthaltende Fraktion als auch die iso-Butyraldehyd enthaltende Fraktion jeweils einer Hydrierung und destillativen Auftrennung nach dem erfindungsgemäßen Verfahren zu unterziehen. Dabei kann entweder nur bei der Hydrierung und Destillation des n-Butyraldehyds oder nur bei der Hydrierung und Destillation des iso-Butyraldehyds ein Strom S1) und/oder ein Strom S2) entnommen werden, oder es wird sowohl bei der Hydrierung und Destillation des n-Butyraldehyds als auch bei der Hydrierung und Destillation des iso-Butyraldehyds ein Strom S1) und/oder ein Strom S2) entnommen. Die Ströme S1) und S2) können unabhängig voneinander auf gleiche oder verschiedene zu erwärmende Stoffströme übertragen werden. In einer speziellen Ausführungsform werden alle entnommenen Ströme S1) und S2) zur Erwärmung des destillativ aufzutrennenden Aldehydgemischs eingesetzt.

In einer weiteren bevorzugten Ausführungsform wird die Hauptmenge der n-Butyraldehyd enthaltenden Fraktion in zwei Teilfraktionen aufgeteilt, die erste Teilfraktion einer Hydrierung und Destillation unterzogen, die zweite Teilfraktion einer Enalisierungsreaktion unterzogen, das bei der Enalisierungsreaktion erhaltene Produkt katalytisch hydriert, bei der Hydrierung des Produkts der Enalisierungsreaktion ein flüssiger Strom S3) entnommen, diesem Strom S3) Wärme durch indirekten Wärmeaustausch entzogen und bei der destillativen Auftrennung des n-/iso-Butyraldehydgemischs eingesetzt. In dieser speziellen Ausführung wird somit die Hydrierwärme aus der 2-Ethylhexenal-Hydrierung und die Hydrierwärme aus den Butanolhydrierungen an die Aldehydtrennkolonne abgegeben. Die 2-Ethylhexenal-Hydrierung wird bevorzugt mit drei in Serie geschalteten Hydrierreaktoren durchgeführt, die alle drei in dem erfindungsgemäßen Wärmeverbund liegen.

Die destillative Auftrennung des Aldehydgemischs kann prinzipiell nach üblichen, dem Fachmann bekannten Destillationsverfahren erfolgen. Geeignete Vorrichtungen für die destillative Aufarbeitung umfassen Destillationskolonnen, wie Bodenkolonnen, die mit Glocken, Siebplatten, Siebböden, Packungen, Einbauten, Ventilen, Seitenabzügen, etc. versehen sein können. Geeignet sind speziell thermisch gekoppelte Kolonnen und Trennwandkolonnen, die mit Seitenabzügen, Rückführungen, etc. versehen sein können. Zur Destillation kann eine Kombination aus zwei oder mehr als zwei Destillationskolonnen eingesetzt werden. Geeignet sind weiterhin Verdampfer, wie Dünnschichtverdampfer, Fallfilmverdampfer, Sambay-Verdampfer, etc. und Kombinationen davon.

In einer speziellen Ausführung wird ein n-/iso-Butyraldehyd enthaltendes Gemisch einer destillativen Auftrennung unterzogen. Speziell handelt es sich um ein rohes n-/iso-Butyraldehyd enthaltendes Gemisch aus der Propen-Hydroformylierung, das eine Hauptmenge an Butyraldehyden und gegebenenfalls zusätzlich weitere Komponenten enthalten kann. Bevorzugt weist das rohe Butyraldehyde enthaltende Gemisch einen Anteil an n- und iso-Butyraldehyd von wenigstens 80 Gew.-%, bevorzugt von wenigstens 90 Gew.-%, insbesondere von wenigstens 95 Gew.-%, bezogen auf das Gesamtgewicht des Gemischs, auf. Die weiteren Komponenten umfassen Butanole und hochsiedende Komponenten, z. B. Produkte der Aldolkondensation.

Ein speziell geeignetes Verfahren zur destillativen Auftrennung eines flüssigen Rohaldehydgemisches ist in der WO 00/58255 (BASF) beschrieben, worauf hier in vollem Umfang Bezug genommen wird.

Danach wird ein Rohaldehydgemisch, das einen geradkettigen und mindestens einen verzweigten Aldehyd enthält, einer destillativen Auftrennung unterzogen, wobei man:
A) das Rohaldehydgemisch in den mittleren Bereich einer ersten Destillationskolonne mit mehreren Trennstufen einspeist und darin eine Auftrennung vornimmt in
   i) einen ersten Aldehydproduktstrom, der über oder nahe dem Kopf der Destillationskolonne abgenommen wird und im Wesentlichen reinen verzweigten Aldehyd umfasst,
   ii) einen zweiten Aldehydproduktstrom, der direkt oberhalb des Verdampfers oder darüber im Bereich der ersten 20 % der Gesamttrennstufen abgenommen wird und der im Wesentlichen reinen geradkettigen Aldehyd umfasst, und
   iii) einen weiteren Produktstrom, der Hochsieder und geradkettigen Aldehyd umfasst und
B) den weiteren Produktstrom in eine zweite Destillationskolonne leitet und darin in einen Aldehydproduktstrom, der im Wesentlichen gereinigten geradkettigen Aldehyd umfasst und über Kopf oder nahe am Kopf der Destillationskolonne abgenommen wird, und in einen Hochsiederstrom aufgetrennt wird.

Bevorzugt wird der in der zweiten Destillationskolonne gewonnene Aldehydproduktstrom in die erste Destillationskolonne zurückgeführt.

Bevorzugt wird der in der zweiten Destillationskolonne erhaltene Produktstrom, der die höher als der geradkettige Aldehyd siedenden Bestandteile umfasst, am Sumpf der zweiten Destillationskolonne abgezogen. Dieser Strom kann einer thermischen Verwertung zugeführt werden. Die dabei erzielte Wärmemenge kann z. B. zur Dampferzeugung genutzt und in einem wärmeverbrauchenden Schritt des erfindungsgemäßen Verfahrens und speziell bei der Erwärmung des destillativ aufzutrennenden Aldehydgemischs eingesetzt werden.

Die destillative Auftrennung eines Aldehydgemischs, das wenigstens zwei C₃-C₂₀-Aldehyde gleicher Kohlenstoffatomanzahl aufweist und speziell die destillative Auftrennung eines n-/iso-Butyraldehydgemischs ist einer der wesentlichen wärmeverbrauchenden Schritte des erfindungsgemäßen Verfahrens. Bevorzugt wird daher Wärme aus den Strömen S1) und/oder S2) auf das aufzutrennende Aldehydgemisch übertragen. Dazu kann ein Strom S1) oder S2) durch einen Wärmetauscher der Trennkolonne geführt werden, der als Verdampfer dient. In einer geeigneten Ausführungsform werden mehrere Ströme S1) und/oder S2) zur Erwärmung des Aldehydgemischs eingesetzt. Dabei kann es sich z. B. um zwei oder mehr als zwei Ströme S1) aus verschiedenen Hydrierungsrektionen handeln, z. B. der Hydrierung von n-Butyraldehyd und der Hydrierung von iso-Butyraldehyd. Des Weiteren kann es sich z. B. um zwei oder mehr als zwei Ströme S2) aus verschiedenen Destillationen handeln, z. B. der Destillation von n-Butanol und der Destillation von iso-Butanol. Weiterhin kann auch wenigstens ein Strom S1) und wenigstens ein Strom S2) zur Erwärmung des Aldehydgemischs eingesetzt werden. Werden zur Erwärmung des Aldehydgemischs mehrere Ströme eingesetzt, so kann dies in einem oder in mehreren baulich getrennt ausgeführten Wärmetauschern erfolgen. Die räumliche Anordnung und Gestaltung der Austauschflächen mit den einzelnen Strömen S1) und S2) und ihre Abfolge innerhalb des Wärmeaustauschers (bzw. beim Einsatz mehrerer Wärmetauscher, deren Auslegung und Reihenfolge) erfolgt unter anderem nach Maßgabe der Temperaturdifferenz zwischen dem zum Wärmeaustausch eingesetzten Strom S1) oder S2) und dem Aldehydstrom.

Werden zur destillativen Auftrennung des Aldehydgemischs mehrere Destillationskolonnen eingesetzt, so können die Ströme S1) und S2) zu Erwärmung des Eduktstroms an jeder der Destillationskolonnen eingesetzt werden. Mehrere Ströme S1) und S2) können bei der Erwärmung des Eduktstroms nur einer oder von mehreren der Destillationskolonnen eingesetzt werden.

Um die zur destillativen Auftrennung erforderliche Energie aufzubringen, kann jede Destillationskolonne zusätzlich mit einer konventionellen Heizung, z. B. mittels Dampf, ausgestattet sein.

### Hydrierung

Bei der Hydrierung liegen der C₃-C₂₀-Aldehyd und der resultierende C₃-C₂₀-Alkohol vorzugsweise in flüssiger Phase vor.

Die Temperatur bei der Hydrierung beträgt vorzugsweise 50 bis 300 °C, insbesondere 100 bis 250 °C.

Der Reaktionsdruck bei der Hydrierung beträgt vorzugsweise 5 bar bis 300 bar, insbesondere 10 bis 150 bar.

Das zur Hydrierung eingesetzte wasserstoffhaltige Gas enthält vorzugsweise mehr als 80 mol-% Wasserstoff. Es besteht insbesondere im Wesentlichen aus Wasserstoff. Das wasserstoffhaltige Gas kann im Gleich- oder Gegenstrom zum Hydrierzulauf geführt werden. Es wird vorzugsweise im Gleichstrom geführt. Die Menge des zugeführten wasserstoffhaltigen Gases wird zweckmäßigerweise so bemessen, dass das 1,0-bis 1,15-fache der stöchiometrisch erforderlichen Wasserstoffmenge zur Verfügung steht.

Als Hydrierkatalysator werden die üblicherweise zur Hydrierung von Aldehyden zu Alkoholen herangezogenen Katalysatoren verwendet. Die Art des verwendeten Katalysators ist nicht Gegenstand der vorliegenden Erfindung; die vorteilhaften Effekte, die durch das erfindungsgemäße Verfahren erzielt werden, sind von der Art des verwendeten Hydrierkatalysators im Allgemeinen unabhängig. Dementsprechend kann im erfindungsgemäßen Verfahren eine Vielzahl von Hydrierkatalysatoren verwendet werden, beispielsweise metallhaltige Trägerkatalysatoren mit Metallen der I., VI und/oder VIII. Nebengruppe des Periodensystems als katalytisch aktiven Komponenten, insbesondere Trägerkatalysatoren mit Rhenium, Platin, Palladium, Rhodium und/oder Ruthenium als katalytisch aktiven Komponenten und Trägermaterialien wie Aluminiumoxid, Titandioxid, Siliciumdioxid, Zirkondioxid, Bariumsulfat; oder Fällungskatalysatoren, die mindestens ein Element aus der I., VI., VII. und/oder VIII. Nebengruppe des Periodensystems enthalten, beispielsweise solche Katalysatoren, wie sie in der DE-A 32 28 881, DE-A 26 28 987 und DE-A 24 45 303 beschrieben sind. Bevorzugt als Hydrierkatalysatoren sind modifizierte Adkins-Katalysatoren, wie sie in J. Am. Chem. Soc. 51, 2430 (1929) und J. Am. Chem. Soc. 54,4678 (1932) beschrieben sind. Ein bevorzugter Hydrierkatalysator weist im nicht reduzierten Zustand 35 Gew.-% Kupfer, berechnet als Cu, 31 Gew.-% Chrom, berechnet als Cr, 2,0 Gew.-% Barium, berechnet als Ba und 2,5 Gew.-% Mangan, berechnet als Mn, auf. Bevorzugt als Hydrierkatalysatoren sind weiterhin Katalysatoren gemäß DE-A 26 28 98. Ein bevorzugter Hydrierkatalysator weist im nicht reduzierten Zustand 24 Gew.-% Nickel, berechnet als NiO, 8 Gew.-% Kupfer, berechnet als CuO, 2,0 Gew.-% Mangan, berechnet als MnO, auf 66 Gew.-% SiO₂ als Trägermaterial auf. Die Katalysatoren liegen vorzugsweise in körniger Form vor und weisen im Allgemeinen eine Teilchengröße von 3 bis 10 mm auf.

Die Hydrierung wird bevorzugt kontinuierlich durchgeführt. Die Hydrierzone kann aus einem einzelnen Reaktor oder aus mehreren Hydrierreaktoren bestehen. In einer speziellen Ausführung des erfindungsgemäßen Verfahrens wird der Hydrierzulauf einer kontinuierlichen Hydrierung in wenigstens zwei (z. B. zwei, drei oder mehr als drei) hintereinander geschalteten Hydrierreaktoren unterzogen. Bevorzugt erfolgt die Hydrierung in einer Kombination von zwei Hydrierreaktoren. Dies ermöglicht eine besonders vorteilhafte Abführung der entstehenden Hydrierwärme. Dies ermöglicht weiterhin eine möglichst vollständige Hydrierung der eingesetzten Aldehyde bei gleichzeitig guter Selektivität bezüglich des gewünschten Alkohols.

Erfolgt die Hydrierung kontinuierlich in wenigstens zwei hintereinander (in Reihe) geschalteten Hydrierreaktoren, so kann jeder Reaktor eine oder mehrere Reaktionszonen innerhalb des Reaktors aufweisen. Bei den Reaktoren kann es sich um gleiche oder verschiedene Reaktoren handeln. Diese können z. B. jeweils gleiche oder verschiedene Vermischungscharakteristiken aufweisen und/oder durch Einbauten ein- oder mehrfach unterteilt sein. Geeignete druckfeste Reaktoren für die Hydrierung sind dem Fachmann bekannt. Dazu zählen die allgemein üblichen Reaktoren für Gas-Flüssig-Reaktionen, wie z. B. Rohrreaktoren, Rohrbündelreaktoren, Gasumlaufreaktoren, Blasensäulen, Schlaufenapparate, Rührkessel (die auch als Rührkesselkaskaden ausgestaltet sein können), Air-Lift-Reaktoren etc.

Die verschiedenen Reaktoren können in Festbett- oder Suspensionsfahrweise betrieben werden. Die Festbettfahrweise ist dabei bevorzugt. Die Katalysatoren können in einem oder mehreren Betten im Reaktor angeordnet sein. In den verschiedenen Betten eines Reaktors bzw. den verschiedenen Reaktoren einer Reaktorkaskade können unterschiedliche Katalysatoren verwendet werden.

Erfolgt die Hydrierung in wenigstens zwei hintereinander geschalteten Hydrierreaktoren, so verfügt der erste Hydrierreaktor vorzugsweise über einen in einem externen Kreislauf geführten Strom (externer Umlaufstrom, Flüssigkeitsumlauf). Vorzugsweise erfolgt die Umsetzung in dem letzten der Reihe geschalteten Reaktoren adiabatisch. Dieser Begriff wird im Rahmen der vorliegenden Erfindung im technischen und nicht im physiko-chemischen Sinne verstanden. So erfährt das Reaktionsgemisch beim Strömen durch den letzten Reaktor auf Grund der exothermen Hydrierungsreaktion in der Regel eine Temperaturerhöhung. Unter adiabatischer Reaktionsführung wird eine Vorgehensweise verstanden, bei der die bei der Hydrierung freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem letzten Reaktor abgeführt, abgesehen von einem Restanteil, der durch natürliche Wärmeleitung und Wärmeabstrahlung vom Reaktor an die Umgebung abgegeben wird. Vorzugsweise wird der letzte Reaktor im geraden Durchgang betrieben.

In einer bevorzugten Ausführung werden zur Hydrierung zwei hintereinander geschaltete Festbettreaktoren eingesetzt. Vorzugsweise verfügt der erste Hydrierreaktor dann über einen in einem externen Kreislauf geführten Strom. Des Weiteren wird die Umsetzung im zweiten Reaktor vorzugsweise adiabatisch durchgeführt. Der zweite Reaktor wird vorzugsweise im geraden Durchgang betrieben.

Eine andere bevorzugte Variante ist die Durchführung der 2-Ethylhexenal-Hydrierung mit drei in Serie geschalteten Hydrierreaktoren in einem gemeinsamen Wälzkreis ohne adiabatischen Nachreaktor.

Erfindungsgemäß wird entweder aus der Hydrierzone (= Strom S1)) oder am Kopf einer Destillationskolonne der folgenden Alkoholdestillation (= Strom S2)) oder aus der Hydrierzone und der Destillationskolonne ein heißer Strom entnommen, diesem Wärme durch indirekten Wärmetausch entzogen und auf einen zu erwärmenden Stoffstrom übertragen.

Der Strom S1) und der Austrag für die Destillation können aus der Hydrierzone separat oder in einem gemeinsamen Strom entnommen werden.

In einer bevorzugten Ausführung werden zur Hydrierung zwei hintereinander geschaltete Reaktoren eingesetzt. Bevorzugt wird dann aus dem ersten Reaktor der Strom S1) und der Feed für den zweiten Reaktor in einem gemeinsamen Strom entnommen. Bevorzugt wird dem gemeinsamen Strom zuerst Wärme entzogen. Anschließend wird der gemeinsame Strom dann geteilt, ein erster Teilstrom in den ersten Reaktor zurückgeführt und ein zweiter Teilstrom als Feed dem zweiten Reaktor zugeführt. Das Verhältnis von zurückgeführtem Strom zu dem zweiten Reaktor zugeführten Strom beträgt vorzugsweise 50 : 1 bis 5 : 1. Gewünschtenfalls kann auch dem zweiten Reaktor ein Strom S1) entnommen und diesem Wärme entzogen werden. Bevorzugt wird dann aus dem zweiten Reaktor der Strom S1) und der Austrag für die Destillation in einem gemeinsamen Strom entnommen. In einer speziellen Ausführung wird dem zweiten Reaktor kein Strom S1) entnommen.

Bei der Hydrierung nach dem erfindungsgemäßen Verfahren werden die Aldehyde in hohen Ausbeuten und mit hoher Selektivität in die entsprechenden Alkohole überführt.

Der Hydrieraustrag besteht im Wesentlichen aus den C₃-C₂₀-Alkoholen, die den eingesetzten C₃-C₂₀-Aldehyden entsprechen. Weitere Bestandteile sind unter Anderem Wasser, Di-(C₃-C₂₀-Alkyl)ether, Hochsieder (z. B. aus Acetalisierung, Aldolisierung, Tischtschenko-Reaktion), etc. Im Folgenden wird bezüglich der weiteren Bestandteile zwischen Niedrigsiedern (d. h. Komponenten, die leichter flüchtig sind als der bei der Hydrierung als Hauptprodukt erhaltene C₃-C₂₀-Alkohol) und Hochsiedern (d. h. Komponenten, die schwerer flüchtig sind als der bei der Hydrierung als Hauptprodukt erhaltene C₃-C₂₀-Alkohol) unterschieden.

### Alkoholdestillation

Der Hydrieraustrag kann anschließend nach üblichen, dem Fachmann bekannten Destillationsverfahren aufgearbeitet werden.

Geeignete Vorrichtungen für die destillative Aufarbeitung umfassen Destillationskolonnen, wie Bodenkolonnen, die mit Glocken, Siebplatten, Siebböden, Packungen, Einbauten, Ventilen, Seitenabzügen, etc. versehen sein können. Geeignet sind speziell Trennwandkolonnen, die mit Seitenabzügen, Rückführungen, etc. versehen sein können. Zur Destillation kann eine Kombination aus zwei oder mehr als zwei Destillationskolonnen eingesetzt werden. Geeignet sind weiterhin Verdampfer, wie Dünnschichtverdampfer, Fallfilmverdampfer, Sambay-Verdampfer, etc. und Kombinationen davon.

In einer bevorzugten Ausführung wird der Austrag aus der Hydrierzone zunächst einer Entgasung unterzogen. Dazu kann der Austrag, gegebenenfalls nach Kühlung, in einen geeigneten Behälter überführt und die Gasphase abgetrennt werden. Diese kann z. B. der thermischen Verwertung zugeführt werden. Der entgaste Hydrieraustrag wird dann zur destillativen Auftrennung eingesetzt.

In einer speziellen Ausführung erfolgt die destillative Auftrennung durch eine Rektifikation, bei der man
i) den Austrag aus der Hydrierzone, gegebenenfalls nach Abtrennung gasförmiger Komponenten, seitlich in eine erste Destillationskolonne einführt,
ii) am Kopf der ersten Destillationskolonne die Niedrigsieder abzieht,
iii) den Sumpf der ersten Destillationskolonne seitlich in eine zweite Destillationskolonne einführt,
iv) am Kopf der zweiten Destillationskolonne einen gasförmigen Austrag entnimmt, diesem als Strom S2) die Wärme entzieht, wobei eine Phasentrennung in eine Gasphase und eine Flüssigphase erfolgt, die Gasphase austrägt und die Flüssigphase teilweise im Kopfbereich in die zweite Destillationskolonne zurückführt und teilweise seitlich in die erste Destillationskolonne einspeist,
v) der zweiten Destillationskolonne seitlich oberhalb der Einspeisung des Sumpfes der ersten Destillationskolonne und unterhalb des Kopfes den gereinigten C₃-C₂₀-Alkohol entnimmt,
vi) am Sumpf der zweiten Destillationskolonne eine die Hochsieder enthaltene Fraktion entnimmt,
vii) gegebenenfalls den Sumpf der zweiten Destillationskolonne einer weiteren Auftrennung unterzieht.

### Herstellung von 2-Ethylhexanol

In einer speziellen Ausführungsform des erfindungsgemäßen Verfahrens wird eine n-Butyraldehyd enthaltende Fraktion einer Enalisierungsreaktion unterzogen, das bei der Enalisierungsreaktion erhaltene Produktgemisch katalytisch hydriert und das Hydrierprodukt einer Destillation unter Erhalt einer an 2-Ethylhexanol angereicherten Fraktion unterzogen. Derartige Verfahren sind dem Fachmann bekannt und z. B. in der US 5,227,544 beschrieben, auf die hier in vollem Umfang Bezug genommen wird.

Die Erfindung wird anhand der folgenden, nicht einschränkenden Beispiele näher erläutert.

### Beispiele:

### Vergleichsbeispiel 1 (Hydrierung von n-Butyraldehyd):

Zur Hydrierung wurde ein 40 m³ Reaktor eingesetzt, dessen Zulauf aus flüssigem n-Butyraldehyd und reinem Wasserstoff besteht. Der n-Butyraldehyd wurde in einer Destillationskolonne durch die Auftrennung eines Rohaldehydgemischs in die Reinkomponenten n-Butyraldehyd und iso-Butyraldehyd gewonnen. Der Dampfverbrauch der Butyraldehyd-Kolonne beträgt 20 bis 30 t/h. Dem Hydrierreaktor wurden kontinuierlich 10 t/h n-Butyraldehyd und 400 kg/h Wasserstoff als Einsatzstoffe zugeführt. Die Kühlung des Hydrierreaktors erfolgte in einem äußeren Kühlkreis, über den die Reaktionswärme der Hydrierung an Luft und Kühlwasser abgegeben wird. Die Temperatur des Ausschleusstroms beträgt am Ausgang des Reaktors 135 °C und beim Wiedereintritt in den Reaktor 115 °C.

### Beispiel 2:

In einem Reaktor gemäß Beispiel 1 wurde die Reaktionswärme der n-Butyraldehydhydrierung als eine der Energiequellen für die Butyraldehyd-Trennkolonne genutzt, indem der äußere Kühlkreislauf durch einen der Verdampfer der Trennkolonne geführt wurde. Die Einsparung an Dampf beträgt dadurch 4,2 t/h.

### Vergleichsbeispiel 3 (Hydrierung von 2-Ethylhexenal):

Zur Hydrierung wurde ein Reaktorsystem eingesetzt, dessen Zulauf aus 10 t/h flüssigem 2-Ethylhexenal und 400 reinem Wasserstoff besteht. Die Kühlung des Hydrierreaktors erfolgte in einem äußeren Kühlkreis, über den die Reaktionswärme der Hydrierung an Luft oder Kühlwasser abgegeben wird. Die Temperatur des Ausschleusstroms beträgt am Ausgang des Reaktors 170 °C und beim Wiedereintritt in den Reaktor 122 °C.

### Beispiel 4:

Die Reaktion aus Vergleichsbeispiel 3 wurde in einem Reaktor gemäß Beispiel 1 durchgeführt und die Reaktionswärme der Hydrierung von 2-Ethylhexenal als eine der Energiequellen für die Butyraldehyd-Trennkolonne genutzt, indem der äußere Kühlkreislauf durch einen der Verdampfer der Trennkolonne geführt wird. Die Einsparung an Dampf beträgt dadurch 5,5 t/h.

### Vergleichsbeispiel 5 (Reindestillation von n-Butanol):

Zur Produktion von reinem n-Butanol wird eine Destillationskolonne eingesetzt, deren Zulauf aus einem bereits von den Leichtsiedern befreiten Rohalkohol besteht. Für einen Zulauf von 10 t/h benötigt man typischerweise einen Dampfstrom von 4,7 t/h. Die der Kolonne damit zugeführte Energiemenge wird ihr im Wesentlichen über den Kopfkondensator wieder entzogen und an ein Kühlmedium abgegeben. Je nach Ausführung der Kolonne kann dies mit Hilfe eines Luftkühlers oder eines Wasserkühlers erfolgen.

### Beispiel 6:

An einer Destillationskolonne gemäß Beispiel 5 wird die Kondensationswärme der n-Butanoldestillation als eine der Energiequellen für die Butyraldehyd-Trennkolonne genutzt, indem die Brüden der Destillationskolonne direkt in einen der Verdampfer der Trennkolonne geführt werden und dort kondensieren. Die Einsparung an Dampf beträgt dadurch 4,5 t/h.

## Patentansprüche

1. Verfahren zur Wärmeintegration bei der Herstellung von gesättigten C₃-C₂₀-Alkoholen, bei dem man einen Hydrierzulauf, der wenigstens einen C₃-C₂₀-Aldehyd enthält, in einer Hydrierzone in Gegenwart eines wasserstoffhaltigen Gases hydriert, aus der Hydrierzone einen Austrag entnimmt und einer Destillation in wenigstens einer Destillationskolonne unterzieht, wobei eine an gesättigten C₃-C₂₀-Alkoholen angereicherte Fraktion erhalten wird, **dadurch gekennzeichnet, dass** man
- aus der Hydrierzone einen flüssigen Strom S1) entnimmt, diesem Strom Wärme entzieht und ihn anschließend in die Hydrierungszone zurückführt, und/oder
- am Kopf wenigstens einer Destillationskolonne einen gasförmigen Strom S2) entnimmt, diesem Strom Wärme entzieht und ihn gegebenenfalls anschließend in die Destillation zurückführt,
wobei die Wärme dem Strom S1) und/oder dem Strom S2) durch indirekten Wärmeaustausch entzogen und ohne den Einsatz eines Hilfsmediums zur Wärmeübertragung auf einen zu erwärmenden Stoffstrom übertragen wird,
wobei man ein Aldehydgemisch, das wenigstens zwei C₃-C₂₀-Aldehyde gleicher Kohlenstoffatomanzahl aufweist, einer destillativen Auftrennung unter Erhalt einer Fraktion unterzieht, welche die Hauptmenge eines der C₃-C₂₀-Aldehyde enthält und diese Fraktion als Hydrierzulauf einsetzt, und
man die dem Strom S1) und/oder die dem Strom S2) entzogene Wärme zur Erwärmung des destillativ aufzutrennenden Aldehydgemischs einsetzt.

2. Verfahren nach einem der vorhergehenden Ansprüche, **dadurch gekennzeichnet, dass** der C₃-C₂₀-Aldehyd ausgewählt ist unter Propionaldehyd, n-Butyraldehyd, iso-Butyraldehyd, Pentanal, Hexanal, Heptanal, 2-Ethylhexanal, 2-Ethylhexenal, Nonanal, Nonenal, Decanal, Decenal sowie den Hydroformylierungsprodukten von Trimerpropylen, Tetramerpropylen, Dimerbuten oder Trimerbuten.

3. Verfahren nach einem der vorhergehenden Ansprüche, **dadurch gekennzeichnet, dass** der C₃-C₂₀-Aldehyd ausgewählt unter n-Butyraldehyd, iso-Butyraldehyd und Mischungen davon.

4. Verfahren nach einem der vorhergehenden Ansprüche, **dadurch gekennzeichnet, dass** die Bereitstellung der C₃-C₂₀-Aldehyde die Hydroformylierung eines Olefin-Ausgangsmaterials umfasst.

5. Verfahren nach einem der vorhergehenden Ansprüche, **dadurch gekennzeichnet, dass** man ein n-/iso-Butyraldehydgemisch einer destillativen Auftrennung unter Erhalt einer die Hauptmenge des n-Butyraldehyds enthaltenden Fraktion und einer die Hauptmenge des iso-Butyraldehyds enthaltenden Fraktion unterzieht und die die Hauptmenge des n-Butyraldehyds enthaltende Fraktion und/oder die die Hauptmenge des iso-Butyraldehyds enthaltende Fraktion als Hydrierzulauf einsetzt.

6. Verfahren nach Anspruch 5, **dadurch gekennzeichnet, dass** man die die Hauptmenge des n-Butyraldehyd enthaltende Fraktion in zwei Teilfraktionen aufteilt, die erste Teilfraktion in einer Hydrierzone in Gegenwart eines wasserstoffhaltigen Gases hydriert, aus der Hydrierzone einen Austrag entnimmt und einer Destillation in wenigstens einer Destillationskolonne unterzieht, wobei eine an n-Butanol angereicherte Fraktion erhalten wird, und die zweite Teilfraktion einer Enalisierungsreaktion unterzieht, das bei der Enalisierungsreaktion erhaltene Produktgemisch katalytisch hydriert und das Hydrierprodukt einer Destillation unter Erhalt einer an 2-Ethylhexanol angereicherten Fraktion unterzieht.

7. Verfahren nach Anspruch 6, **dadurch gekennzeichnet, dass** man bei der Hydrierung des Produkts der Enalisierungsreaktion einen flüssigen Strom S3) entnimmt, diesem Strom S3) Wärme durch indirekten Wärmeaustausch entzieht und ohne den Einsatz eines Hilfsmediums zur Wärmeübertragung auf einen zu erwärmenden Stoffstrom überträgt und den Strom S3) anschließend in die Hydrierung des Produkts der Enalisierungsreaktion zurückführt.

8. Verfahren nach einem der vorhergehenden Ansprüche, **dadurch gekennzeichnet, dass** man die dem Strom S3) entzogene Wärme zur Erwärmung eines Stoffstroms bei der Herstellung der gesättigten C₃-C₂₀-Alkohole einsetzt.

9. Verfahren nach einem der Ansprüche 4 bis 6, **dadurch gekennzeichnet, dass** man die dem Strom S3) entzogene Wärme zur Erwärmung des destillativ aufzutrennenden Aldehydgemischs einsetzt.

10. Verfahren nach einem der vorhergehenden Ansprüche, **dadurch gekennzeichnet, dass** man den Strom S1) und/oder den Strom S3) ohne Abtrennung einer stofflichen Komponente in die Hydrierzone zurückführt.

11. Verfahren nach einem der vorhergehenden Ansprüche, **dadurch gekennzeichnet, dass** der Strom S2) beim Wärmeaustausch kondensiert wird.

12. Verfahren zur Wärmeintegration bei der Herstellung von n-Butanol und/oder iso-Butanol, bei dem man
a) ein n-/iso-Butyraldehydgemisch einer destillativen Auftrennung unter Erhalt einer die Hauptmenge des n-Butyraldehyd enthaltenden Fraktion und einer die Hauptmenge des iso-Butyraldehyd enthaltenden Fraktion unterzieht,
b1) den die Hauptmenge des n-Butyraldehyd enthaltenden Strom aus Schritt a) in einer Hydrierungszone H1) in Gegenwart von Wasserstoff einer Hydrierung unterzieht,
c1) aus der Hydrierungszone H1) einen flüssigen Strom S1c1) entnimmt, diesem Strom Wärme entzieht, indem man ihn gemeinsam mit dem in Schritt a) aufzutrennenden n-/iso-Butyraldehydgemisch durch einen Wärmeaustauscher führt und ihn anschließend in die Hydrierzonezone H1) zurückführt,
d1) aus der Hydrierungszone H1) einen Austrag entnimmt und einer Destillation in wenigstens einer Destillationskolonne unterzieht, wobei eine an n-Butanol angereicherte Fraktion erhalten wird,
e1) am Kopf wenigstens einer der Destillationskolonnen einen gasförmigen Strom S2e1) entnimmt, diesem Strom Wärme entzieht, indem man ihn gemeinsam mit dem in Schritt a) aufzutrennenden n-/iso-Butyraldehydgemisch durch einen Wärmeaustauscher führt und ihn gegebenenfalls anschließend in die Destillation zurückführt,
und/oder
b2) den die Hauptmenge des iso-Butyraldehyd enthaltenden Strom aus Schritt a) in einer Hydrierungszone H2) in Gegenwart von Wasserstoff einer Hydrierung unterzieht,
c2) aus der Hydrierungszone H2) einen flüssigen Strom S1c2) entnimmt, diesem Strom Wärme entzieht, indem man ihn gemeinsam mit dem in Schritt a) aufzutrennenden n-/iso-Butyraldehydgemisch durch einen Wärmeaustauscher führt und ihn anschließend in die Hydrierzonezone H2) zurückführt,
d2) aus der Hydrierungszone H2) einen Austrag entnimmt und einer Destillation in wenigstens einer Destillationskolonne unterzieht, wobei eine an iso-Butanol angereicherte Fraktion erhalten wird,
e2) am Kopf wenigstens einer der Destillationskolonnen einen gasförmigen Strom S2e2) entnimmt, diesem Strom Wärme entzieht, indem man ihn gemeinsam mit dem in Schritt a) aufzutrennenden n-/iso-Butyraldehydgemisch durch einen Wärmeaustauscher führt und ihn gegebenenfalls anschließend in die Destillation zurückführt,
mit der Maßgabe, dass wenigstens einer der Schritte c1), e1), c2) oder e2) zwingend vorgesehen ist.

13. Verfahren nach Anspruch 12, **dadurch gekennzeichnet, dass** man in Schritt a) die die Hauptmenge des n-Butyraldehyd enthaltende Fraktion zumindest teilweise einer Enalisierungsreaktion unterzieht, das bei der Enalisierungsreaktion erhaltene Produktgemisch katalytisch hydriert und das Hydrierprodukt einer Destillation unter Erhalt einer an 2-Ethylhexanol angereicherten Fraktion unterzieht.

14. Verfahren nach Anspruch 13, **dadurch gekennzeichnet, dass** man bei der Hydrierung des Produkts der Enalisierungsreaktion einen flüssigen Strom S3) entnimmt, diesem Strom S3) Wärme entzieht, indem man ihn gemeinsam mit dem in Schritt a) aufzutrennenden n-/iso-Butyraldehydgemisch durch einen Wärmeaustauscher führt und den Strom S3) anschließend in die Hydrierung des Produkts der Enalisierungsreaktion zurückführt.

## Claims

1. A process for heat integration in the preparation of saturated C₃-C₂₀-alcohols, in which a hydrogenation feed comprising at least one C₃-C₂₀-aldehyde is hydrogenated in the presence of a hydrogen-comprising gas in a hydrogenation zone and a discharge is taken off from the hydrogenation zone and subjected to distillation in at least one distillation column to give a fraction which is enriched in saturated C₃-C₂₀-alcohols, wherein
- a liquid stream S1) is taken off from the hydrogenation zone, heat is withdrawn from this stream and the stream is subsequently recirculated to the hydrogenation zone and/or
- a gaseous stream S2) is taken off at the top of at least one distillation column, heat is withdrawn from this stream and the stream is optionally then recirculated to the distillation,
where the heat is withdrawn from the stream S1) and/or the stream S2) by indirect heat exchange and is transferred to a stream to be heated without the use of an auxiliary medium for heat transfer, wherein an aldehyde mixture comprising at least two C₃-C₂₀-aldehydes having the same number of carbon atoms is subjected to fractional distillation to give a fraction comprising the major part of one of the C₃-C₂₀-aldehydes and this fraction is used as hydrogenation feed, and
the heat withdrawn from the stream S1) and/or the heat withdrawn from the stream S2) is used for heating the aldehyde mixture to be fractionally distilled.

2. The process according to the preceding claim, wherein the C₃-C₂₀-aldehyde is selected from among propionaldehyde, n-butyraldehyde, isobutyraldehyde, pentanal, hexanal, heptanal, 2-ethylhexanal, 2-ethylhexenal, nonanal, nonenal, decanal, decenal and the hydroformylation products of propylene trimer, propylene tetramer, butene dimer or butene trimer.

3. The process according to any of the preceding claims, wherein the C₃-C₂₀-aldehyde is selected from among n-butyraldehyde, isobutyraldehyde and mixtures thereof.

4. The process according to any of the preceding claims, wherein the provision of the C₃-C₂₀-aldehydes comprises the hydroformylation of an olefin starting material.

5. The process according to any of the preceding claims, wherein an n-butyraldehyde/isobutyraldehyde mixture is subjected to fractional distillation to give a fraction comprising the major part of the n-butyraldehyde and a fraction comprising the major part of the isobutyraldehyde and the fraction comprising the major part of the n-butyraldehyde and/or the fraction comprising the major part of the isobutyraldehyde is used as hydrogenation feed.

6. The process according to claim 5, wherein the fraction comprising the major part of the n-butyraldehyde is divided into two subfractions, the first subfraction is hydrogenated in the presence of a hydrogen-comprising gas in a hydrogenation zone, a discharge is taken off from the hydrogenation zone and subjected to a distillation in at least one distillation column to give a fraction enriched in n-butanol and the second subfraction is subjected to an enalization reaction, the product mixture obtained in the enalization reaction is catalytically hydrogenated and the hydrogenation product is subjected to a distillation to give a fraction enriched in 2-ethylhexanol.

7. The process as claimed in claim 6, wherein a liquid stream S3) is taken off in the hydrogenation of the product of the enalization reaction, heat is withdrawn from this stream S3) by indirect heat exchange and transferred to a stream to be heated without the use of an auxiliary medium for heat exchange and the stream S3) is subsequently recirculated to the hydrogenation of the product of the enalization reaction.

8. The process according to any of the preceding claims, wherein the heat withdrawn from the stream S3) is used for heating a stream in the preparation of the saturated C₃-C₂₀-alcohols.

9. The process according to any of claims 4 to 6, wherein the heat withdrawn from the stream S3) is used for heating the aldehyde mixture to be fractionally distilled.

10. The process according to any of the preceding claims, wherein the stream S1) and/or the stream S3) is recirculated without removal of a material component to the hydrogenation zone.

11. The process according to any of the preceding claims, wherein the stream S2) is condensed in the heat exchange.

12. A process for heat integration in the preparation of n-butanol and/or isobutanol, wherein
a) an n-butyraldehyde/isobutyraldehyde mixture is subjected to fractional distillation to give a fraction comprising the major part of the n-butyraldehyde and a fraction comprising the major part of the isobutyraldehyde,
b1) the stream comprising the major part of the n-butyraldehyde from step a) is subjected to hydrogenation in the presence of hydrogen in a hydrogenation zone H1),
c1) a liquid stream S1c1) is taken off from the hydrogenation zone H1), heat is withdrawn from this stream by passing it together with the n-butyraldehyde/isobutyraldehyde mixture to be fractionated in step a) through a heat exchanger and subsequently recirculating the stream to the hydrogenation zone H1),
d1) a discharge is taken off from the hydrogenation zone H1) and subjected to distillation in at least one distillation column to give a fraction enriched in n-butanol,
e1) a gaseous stream S2e1) is taken off at the top of at least one of the distillation columns, heat is withdrawn from this stream by passing it together with the n-butyraldehyde/isobutyraldehyde mixture to be fractionated in step a) through a heat exchanger and optionally then recirculating the stream to the distillation,
and/or
b2) the stream comprising the major part of the isobutyraldehyde from step a) is subjected to hydrogenation in the presence of hydrogen in a hydrogenation zone H2),
c2) a liquid stream S1c2) is taken off from the hydrogenation zone H2), heat is withdrawn from this stream by passing it together with the n-butyraldehyde/isobutyraldehyde mixture to be fractionated in step a) through a heat exchanger and subsequently recirculating the stream to the hydrogenation zone H2),
d2) a discharge is taken off from the hydrogenation zone H2) and subjected to distillation in at least one distillation column to give a fraction enriched in isobutanol,
e2) a gaseous stream S2e2) is taken off at the top of at least one of the distillation columns, heat is withdrawn from this stream by passing it together with the n-butyraldehyde/isobutyraldehyde mixture to be fractionated in step a) through a heat exchanger and optionally then recirculating the stream to the distillation,
with the proviso that at least one of the steps c1), e1), c2) or e2) is necessarily provided.

13. The process according to claim 12, wherein, in step a), the fraction comprising the major part of the n-butyraldehyde is at least partly subjected to an enalization reaction, the product mixture obtained in the enalization reaction is catalytically hydrogenated and the hydrogenation product is subjected to a distillation to give a fraction enriched in 2-ethylhexanol.

14. The process according to claim 13, wherein a liquid stream S3) is taken off in the hydrogenation of the product of the enalization reaction, heat is withdrawn from this stream S3) by passing it together with the n-butyraldehyde/isobutyraldehyde mixture to be fractionated in step a) through a heat exchanger and the stream S3) is subsequently recirculated to the hydrogenation of the product of the enalization reaction.

## Revendications

1. Procédé d'intégration thermique lors de la fabrication d'alcools en C₃-C₂₀ saturés, selon lequel une alimentation d'hydrogénation, qui contient au moins un aldéhyde en C₃-C₂₀, est hydrogénée dans une zone d'hydrogénation en présence d'un gaz contenant de l'hydrogène, une sortie est soutirée de la zone d'hydrogénation et soumise à une distillation dans au moins une colonne de distillation, une fraction enrichie en alcools en C₃-C₂₀ saturés étant obtenue, **caractérisé en ce que**
- un courant liquide S1) est soutiré de la zone d'hydrogénation, de la chaleur est extraite de ce courant, puis il est recyclé dans la zone d'hydrogénation, et/ou
- un courant gazeux S2) est soutiré à la tête d'au moins une colonne de distillation, de la chaleur est extraite de ce courant, puis il est éventuellement recyclé dans la distillation,
la chaleur étant extraite du courant S1) et/ou du courant S2) par échange de chaleur indirect et transférée à un courant de matière à chauffer sans utiliser un milieu auxiliaire pour le transfert de chaleur,
un mélange d'aldéhydes, qui comprend au moins deux aldéhydes en C₃-C₂₀ ayant un nombre de carbones identique, étant soumis à une séparation par distillation pour obtenir une fraction qui contient la quantité principale d'un des aldéhydes en C₃-C₂₀ et cette fraction étant utilisé en tant qu'alimentation d'hydrogénation, et
la chaleur extraite du courant S1) et/ou du courant S2) étant utilisée pour le chauffage du mélange d'aldéhydes à séparer par distillation.

2. Procédé selon l'une quelconque des revendications précédentes, **caractérisé en ce que** l'aldéhyde en C₃-C₂₀ est choisi parmi le propionaldéhyde, le n-butyraldéhyde, l'iso-butyraldéhyde, le pentanal, l'hexanal, l'heptanal, le 2-éthylhexanal, le 2-éthylhexénal, le nonanal, le nonénal, le décanal, le décénal, ainsi que les produits d'hydroformylation de propylène trimère, de propylène tétramère, de butène dimère ou de butène trimère.

3. Procédé selon l'une quelconque des revendications précédentes, **caractérisé en ce que** l'aldéhyde en C₃-C₂₀ est choisi parmi le n-butyraldéhyde, l'iso-butyraldéhyde et leurs mélanges.

4. Procédé selon l'une quelconque des revendications précédentes, **caractérisé en ce que** la préparation des aldéhydes en C₃-C₂₀ comprend l'hydroformylation d'un matériau de départ oléfinique.

5. Procédé selon l'une quelconque des revendications précédentes, **caractérisé en ce qu'**un mélange de n-/iso-butyraldéhyde est soumis à une séparation par distillation pour obtenir une fraction contenant la quantité principale du n-butyraldéhyde et une fraction contenant la quantité principale de l'iso-butyraldéhyde, et la fraction contenant la quantité principale du n-butyraldéhyde et/ou la fraction contenant la quantité principale de l'iso-butyraldéhyde sont utilisées en tant qu'alimentation d'hydrogénation.

6. Procédé selon la revendication 5, **caractérisé en ce que** la fraction contenant la quantité principale du n-butyraldéhyde est divisée en deux fractions partielles, la première fraction partielle est hydrogénée dans une zone d'hydrogénation en présence d'un gaz contenant de l'hydrogène, une sortie est soutirée de la zone d'hydrogénation et soumise à une distillation dans au moins une colonne de distillation, une fraction enrichie en n-butanol étant obtenue, et la deuxième fraction partielle est soumise à une réaction d'énalisation, le mélange de produits obtenu lors de la réaction d'énalisation est hydrogéné catalytiquement et le produit d'hydrogénation est soumis à une distillation pour obtenir une fraction enrichie en 2-éthylhexanol.

7. Procédé selon la revendication 6, **caractérisé en ce qu'**un courant liquide S3) est soutiré lors de l'hydrogénation du produit de la réaction d'énalisation, de la chaleur est extraite de ce courant S3) par échange de chaleur indirect et transférée à un courant de matière à chauffer sans utiliser un milieu auxiliaire pour le transfert de chaleur, puis le courant S3) est recyclé dans l'hydrogénation du produit de la réaction d'énalisation.

8. Procédé selon l'une quelconque des revendications précédentes, **caractérisé en ce que** la chaleur extraite du courant S3) est utilisée pour le chauffage d'un courant de matière lors de la fabrication des alcools en C₃-C₂₀ saturés.

9. Procédé selon l'une quelconque des revendications 4 à 6, **caractérisé en ce que** la chaleur extraite du courant S3) est utilisée pour le chauffage du mélange d'aldéhydes à séparer par distillation.

10. Procédé selon l'une quelconque des revendications précédentes, **caractérisé en ce que** le courant S1) et/ou le courant S3) sont recyclés dans la zone d'hydrogénation sans séparation d'un composant matériel.

11. Procédé selon l'une quelconque des revendications précédentes, **caractérisé en ce que** le courant S2) est condensé lors de l'échange de chaleur.

12. Procédé d'intégration thermique lors de la fabrication de n-butanol et/ou d'iso-butanol, selon lequel
a) un mélange de n-/iso-butyraldéhyde est soumis à une séparation par distillation pour obtenir une fraction contenant la quantité principale du n-butyraldéhyde et une fraction contenant la quantité principale de l'iso-butyraldéhyde,
b1) le courant contenant la quantité principale du n-butyraldéhyde de l'étape a) est soumis à une hydrogénation dans une zone d'hydrogénation H1) en présence d'hydrogène,
c1) un courant liquide S1c1) est soutiré de la zone d'hydrogénation H1), de la chaleur est extraite de ce courant par passage de celui-ci conjointement avec le mélange de n-/iso-butyraldéhyde à séparer à l'étape a) dans un échangeur de chaleur, puis il est recyclé dans la zone d'hydrogénation H1),
d1) une sortie est soutirée de la zone d'hydrogénation H1) et soumise à une distillation dans au moins une colonne de distillation, une fraction enrichie en n-butanol étant obtenue,
e1) un courant gazeux S2e1) est soutiré à la tête d'au moins une des colonnes de distillation, de la chaleur est extraite de ce courant par passage de celui-ci conjointement avec le mélange de n-/iso-butyraldéhyde à séparer à l'étape a) dans un échangeur de chaleur, puis il est éventuellement recyclé dans la distillation, et/ou
b2) le courant contenant la quantité principale de l'iso-butyraldéhyde de l'étape a) est soumis à une hydrogénation dans une zone d'hydrogénation H2) en présence d'hydrogène,
c2) un courant liquide S1c2) est soutiré de la zone d'hydrogénation H2), de la chaleur est extraite de ce courant par passage de celui-ci conjointement avec le mélange de n-/iso-butyraldéhyde à séparer à l'étape a) dans un échangeur de chaleur, puis il est recyclé dans la zone d'hydrogénation H2),
d2) une sortie est soutirée de la zone d'hydrogénation H2) et soumise à une distillation dans au moins une colonne de distillation, une fraction enrichie en iso-butanol étant obtenue,
e2) un courant gazeux S2e2) est soutiré à la tête d'au moins une des colonnes de distillation, de la chaleur est extraite de ce courant par passage de celui-ci conjointement avec le mélange de n-/iso-butyraldéhyde à séparer à l'étape a) dans un échangeur de chaleur, puis il est éventuellement recyclé dans la distillation,
à condition qu'au moins une des étapes c1), e1), c2) ou e2) soit obligatoirement prévue.

13. Procédé selon la revendication 12, **caractérisé en ce qu'**à l'étape a), la fraction contenant la quantité principale du n-butyraldéhyde est soumise au moins en partie à une réaction d'énalisation, le mélange de produits obtenu lors de la réaction d'énalisation est hydrogéné catalytiquement et le produit d'hydrogénation est soumis à une distillation pour obtenir une fraction enrichie en 2-éthylhexanol.

14. Procédé selon la revendication 13, **caractérisé en ce qu'**un courant liquide S3) est soutiré lors de l'hydrogénation du produit de la réaction d'énalisation, de la chaleur est extraite de ce courant S3) par passage de celui-ci conjointement avec le mélange de n-/iso-butyraldéhyde à séparer à l'étape a) dans un échangeur de chaleur, puis le courant S3) est recyclé dans l'hydrogénation du produit de la réaction d'énalisation.
